(19) Europäisches Patentamt
European Patent Office
Office européen des brevets

(11) Veröffentlichungsnummer : **0 216 386 B1**

# (12) EUROPÄISCHE PATENTSCHRIFT

(45) Veröffentlichungstag der Patentschrift : **05.06.91 Patentblatt 91/23**

(51) Int. Cl.$^5$ : **C07D 233/64**, C07D 233/68, C07D 233/91

(21) Anmeldenummer : **86113227.2**

(22) Anmeldetag : **25.09.86**

## (54) Verfahren zur Herstellung von Hydroximethylimidazolen.

(30) Priorität : **25.09.85 DE 3534083**

(43) Veröffentlichungstag der Anmeldung : **01.04.87 Patentblatt 87/14**

(45) Bekanntmachung des Hinweises auf die Patenterteilung : **05.06.91 Patentblatt 91/23**

(84) Benannte Vertragsstaaten : **BE CH DE FR GB IT LI**

(56) Entgegenhaltungen : **Keine Entgegenhaltungen**

(73) Patentinhaber : **BASF Aktiengesellschaft**
**Carl-Bosch-Strasse 38**
**W-6700 Ludwigshafen (DE)**

(72) Erfinder : **Graf, Fritz, Dr.**
**Im Rothschild 33**
**W-6720 Speyer (DE)**
Erfinder : **Hupfer, Leopold, Dr.**
**Waltershoehe 3**
**W-6701 Friedelsheim (DE)**

EP 0 216 386 B1

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Diese Erfindung betrifft ein neues Verfahren zur Herstellung von Imidazolen, die an einem C-Atom des Imidazolringes einen Hydroximethylrest tragen, durch Umlagerung von 1-Hydroximethylimidazolen an einem basischen Ionenaustauscher.

4(5)-Methyl-5(4)-hydroximethylimidazol läßt sich z.B. nach dem in Helv. Chim. Acta 31, 38 (1948) beschriebenen Verfahren durch Erhitzen von 4(5)-Methylimidazol mit Formaldehyd im Bombenrohr auf 120°C gewinnen. Aus der EP-PS 4534 und der DE-OS 29 34 925 ist bekannt, daß man 4(5)-Methyl-5(4)-hydroximethylimidazol günstiger herstellen kann, wenn man die Umsetzung von 4(5)-Methylimidazol mit Formaldehyd in wäßrig alkalischem Milieu bei einem pH von 11 bis 13 und Temperaturen von 20 bis 60°C oder in Gegenwart einer starken Base bei 30 bis 95°C vornimmt. Von Nachteil ist bei diesen Verfahren die umständliche Aufarbeitung zur Abtrennung der zur Synthese erforderlichen Basen. Im allgemeinen fällt 4(5)-Methyl-5(4)-hydroximethylimidazol in Form des Hydrochlorids an.

Man kann das Hydrochlorid von 4(5)-Methyl-5(4)-hydroximethylimidazol auch durch Umsetzung von 4(5)-Methylimidazol mit Formaldehyd unter Druck und erhöhten Temperaturen in Gegenwart von Salzsäure herstellen. Nachteilig ist auch hier die umständliche Aufarbeitung. Außerdem benötigt man korrosionsfeste Materialien.

Es wurde nun gefunden, daß man Imidazole, die an einem C-Atom des Imidazolringes einen Hydroximethylrest tragen, erheblich vorteilhafter herstellen kann, wenn man 1-Hydroximethylimidazole, die zumindest in einer der Positionen 2, 4 und 5 des Imidazolinges nicht substituiert sind, mit einem Anionenaustauscher behandelt. Nach dem neuen Verfahren erhält man die Hydroximethylimidazole auf besonders einfache Weise in guter Ausbeute und hoher Reinheit.

Als Ausgangsstoffe sind 1-Hydroximethylimidazole geeignet, die z.B. in 5(4)-Stellung nicht substituiert sind und in den Stellungen 2 und 4 (5) Substituenten, wie Halogenatome oder Alkyl, Hydroxyalkyl, Nitrogruppen oder Phenylreste enthalten können. Als Alkyl- oder Hydroxyalkylreste kommen z.B. Methyl, Ethyl, Propyl, Hexyl, Decyl, Hydroximethyl, Hydroxiethyl und Hydroxypropyl in Betracht. Die Phenylreste können substituiert sein, z.B. durch Halogenatome, wie Fluor, Chlor oder Brom oder durch Nitrogruppen, Methyl- oder Ethylreste.

Beispielsweise seien folgende Ausgangsverbindungen genannt : 1-Hydroximethylimidazol, 4(5)-Methyl-1-hydroximethylimidazol,
2-Methyl-1-hydroximethylimidazol, 4(5)-Nitro-1-hydroximethylimidazol, 2- oder 4(5)-Phenyl-1-hydroximethylimidazol,
2-Isopropyl-1-hydroximethylimidazol, 2, 4(5)-Dimethyl-1-hydroximethylimidazol, 2- oder 4(5)-Ethyl-1-hydroximethylimidazol und 4, 5-Dimethyl-1-hydroxymethylimidazol. Man kann diese Ausgangsverbindungen z.B. nach dem in der DE-OS 28 25 547 beschriebenen Verfahren herstellen.

Nach dem Verfahren der Erfindung, bei dem der Hydroximethylrest an eine der freien Positionen 2, 4 und 5 des Imidazolringes wandert, behandelt man die 1-Hydroximethylimidazole mit dem Anionenaustauscher z.B. 0,1 bis 100 Stunden bei Temperaturen von 0 bis 120°C, vorzugsweise bei 30 bis 70°C. Behandlungszeiten von 1 bis 50 Stunden sind bevorzugt.

Die 1-Hydroximethylimidazole werden bei dem erfindungsgemäßen Verfahren zweckmäßigerweise als Lösung in einem Lösungsmittel eingesetzt, wobei man auch Mischungen von Lösungsmitteln verwenden kann. Geeignete Lösungsmittel sind z.B. Wasser, Alkohole, wie Methanol, Ethanol oder Ketone, wie Aceton oder Methylethylketon. Man verwendet z.B. Lösungen, die einen Gehalt an der Imidazolverbindung von bis zu 85 Gew.%, vorzugsweise zwischen 20 und 80 Gew.%, insbesondere 50 bis 75 Gew.% aufweisen. Vorzugsweise verwendet man Lösungen der 1-Hydroximethylimidazole in Wasser, wie eine 5 bis 85 gewichtsprozentige wäßrige Lösung.

Als Anionenaustauscher sind alle üblichen basischen Ionenaustauscher geeignet, z.B. solche auf Basis Styrol-DVB oder Polyaminkondensat, wie die im Handel unter den Bezeichnungen DOWEX®, AMBERLIT® und LEWATIT® erhältichen Ionenaustauscher. Besonders geeignet sind LEWATIT M 406® und DOWEX MSA®. Der Anionenaustauscher erfährt bei der erfindungsgemäßen Verwendung keine Veränderung und kann beliebig oft eingesetzt werden.

Das erfindungsgemäße Verfahren wird z.B. so durchgeführt, daß man eine Lösung des 1-Hydroximethylimidazols bei Temperaturen von 40 bis 80°C zwei bis fünf Stunden durch ein mit dem Anionenaustauscher gefülltes Rohr leitet. Aus der ablaufenden Reaktionslösung isoliert man das Hydroximethylimidazol auf an sich bekannte Weise, z.B. durch Auskristallisieren der gegebenenfalls durch Eindampfen aufkonzentrierten Reaktionslösung. Durch Auswaschen, z.B. mit Aceton oder durch Umkristallisieren kann das Hydroximethylimidazol in reiner Form erhalten werden.

Die nach dem erfindungsgemäßen Verfahren erhältichen Hydroximethylimidazole sind wichtige Zwischenprodukte, z.B. für die Herstellung von Arzneimitteln.

## Beispiel

Eine Lösung von 112 g 4(5)-Methyl-1-hydroximethylimidazol in 45 ml Wasser wird während drei Stunden bei 60°C durch ein mit 1 000 ml Anionenaustauscher gefülltes Rohr geleitet. Dabei wurde der im

Handel unter der Bezeichnung Lewatit® M 504 erhältliche Anionenaustauscher verwendet. Die ablaufende Reaktionslösung, die 48,34%, entsprechend 75,9 g 4(5)-Methyl-4(5)-hydroximethylimidazol (67,77% Ausbeute) enthält, erstarrt beim Stehen weitgehend. Das auskristallisierte 4(5)-Methyl-5(4)-hydroximethylimidazol wird abgesaugt und anschließend durch Auswaschen mit Aceton von Nebenprodukten befreit; es hat nach dem Trocknen einen Schmelzpunkt von 137 bis 138°C.

Die abgetrennte wäßrige Mutterlauge wird zur Trockne eingeengt und der Rückstand mit dem oben angefallenen Waschaceton behandelt, wobei man 12 g einer zweiten Fraktion 4(5)-Methyl-5(4)-hydroximethylimidazol von etwas geringerer Reinheit erhält.

## Ansprüche

1. Verfahren zur Herstellung von Imidazolen, die an einem C-Atom des Imidazolringes einen Hydroximethylrest enthalten, dadurch gekennzeichnet, daß man 1-Hydroximethylimidazole, die zumindest in einer der Positionen 2, 4 und 5 des Imidazolringes nicht substituiert sind, mit einem Anionenaustauscher behandelt.

2. Verfahren nach Anspruch 1, dadurch gekennzeichnet, daß man die Behandlung bei 0 bis 100°C vornimmt.

3. Verfahren nach Anspruch 1, dadurch gekennzeichnet, daß man das 1-Hydroximethylimidazol als Lösung in einem Lösungsmittel einsetzt.

4. Verfahren nach Anspruch 1, dadurch gekennzeichnet, daß man eine 5 bis 85 gewichtsprozentige wäßrige Lösung der 1-Hydroximethylimidazole mit dem Anionenaustauscher behandelt.

5. Verfahren nach Anspruch 1, dadurch gekennzeichnet, daß man als 1-Hydroximethylimidazole solche der Formel

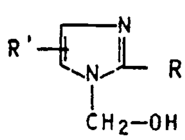

einsetzt, in der R' in der Position 4 oder 5 steht und R und R' Wasserstoffatome, Halogenatome, Alkylgruppen, Hydroxyalkylgruppen, Nitrogruppen oder Phenylresten, die durch Halogenatome, Nitrogruppen, Methyl- oder Ethylreste substituiert sein können.

6. Verfahren nach Anspruch 1, dadurch gekennzeichnet, daß man 4(5)-Methyl-5(4)-hydroximethylimidazol aus 4(5)-Methyl-1-hydroximethylimidazol herstellt.

## Claims

1. A process for the preparation of an imidazole which contains a hydroxymethyl radical on a carbon atom of the imidazole ring, wherein a 1-hydroxymethylimidazole which is unsubstituted in one or more of the positions 2, 4 and 5 of the imidazole ring is treated with an anion exchanger.

2. A process as claimed in claim 1, wherein the treatment is carried out at from 0 to 100°C.

3. A process as claimed in claim 1, wherein the 1-hydroxymethylimidazole is used in the form of a solution in a solvent.

4. A process as claimed in claim 1, wherein a 5-85% strength by weight aqueous solution of the 1-hydroxymethylimidazole is treated with the anion exchanger.

5. A process as claimed in claim 1, wherein the 1-hydroxymethylimidazole used is of the formula

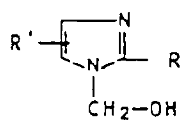

where R' is in the 4 or 5 position and R and R' are each hydrogen, halogen, alkyl, hydroxyalkyl, nitro or phenyl which may be substituted by halogen, nitro, methyl or ethyl.

6. A process as claimed in claim 1, wherein 4(5)-methyl-5(4)-hydroxymethylimidazole is prepared from 4(5)-methyl-1-hydroxymethylimidazole.

## Revendications

1. Procédé de préparation d'imidazoles qui portent un reste hydroxyméthyle sur un atome de carbone du noyau imidazole, caractérisé en ce qu'on traite par un échangeur d'anions des 1-hydroxyméthylimidazoles qui ne sont pas substitués au moins en une des positions 2, 4 et 5 du noyau imidazole

2. Procédé selon la revendication 1, caractérisé en ce qu'on éffectue le traitement à 0-100°C

3. Procédé selon la revendication 1, caractérisé en ce qu'on met en réaction le 1-hydroxymethylimidazole en solution dans un solvant.

4. Procédé selon la revendication 1, caractérisé en ce qu'on traite par l'échangeur d'anions une solution aqueuse à 5-85% en poids de 1-hydroxyméthylimidazole.

5. Procédé selon la revendication 1, caractérisé en ce qu'on met en réaction comme 1-hydroxyméthylimidazole ceux de formule

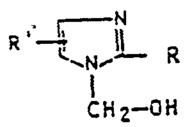

dans laquelle R′ est en position 4 ou 5 et R et R′ sont des atomes d'hydrogène, des atomes d'halogène, des groupements alkyle, des groupements hydroxyalkyle, des groupements nitro ou des restes phényle qui peuvent être substitués par des atomes d'halogène, des groupements nitro ou des restes méthyle ou éthyle.

6. Procédé selon la revendication 1, caractérisé en ce qu'on prépare le 4(5)-méthyl-5(4)-hydro-hydroxyméthylimidazole à partir du 4(5)-méthyl-1-hydroxyméthylimidazole.